# EUROPÄISCHE PATENTSCHRIFT

(11) **EP 0 402 810 B1**
(45) Veröffentlichungstag und Bekanntmachung des Hinweises auf die Patenterteilung: **07.04.1993**
(21) Anmeldenummer: 90110953.8
(22) Anmeldetag: 09.06.1990
(51) Int. Cl.: A61F 2/34

(54) **Implantationssatz**
Implantation set
Ensemble d'implantation

(30) Priorität: 12.06.1989 DE 3918970
(43) Veröffentlichungstag der Anmeldung: 19.12.1990
(73) Patentinhaber: ESKA MEDICAL LÜBECK MEDIZINTECHNIK GmbH & Co., D-23556 Lübeck (DE)
(72) Erfinder: Heinz, Moser, D-6227 Oestrich-Winkel (DE); Gradinger, Reiner, Dr., D-8000 München 2 (DE)
(74) Vertreter: Fuchs Mehler Weiss

(56) Entgegenhaltungen:
- EP-A- 0 121 002
- EP-A- 0 211 169
- EP-A- 0 295 912
- EP-A- 0 314 951
- DE-C- 3 620 460

## Beschreibung

Die Erfindung betrifft einen Implantationssatz, bestehend aus einer in eine Beckenknochenpfanne einpreßbaren, auf ihrer Außenfläche mit einer offenzelligen metallischen Struktur versehenen kalottenförmigen Gelenkpfanne und aus jeweils mindestens einem ersten und zweiten Fixationselement zur Verspannung der Gelenkpfanne in dem Beckenknochen.

Gelenkpfannen für Beckenknochen, die allerdings nicht Bestandteil eines modularen Implantationssatzes sind, sind bekannt beispielsweise aus der DE-OS 16 16 151, der DE-GM 72 40 856 und DE-OS 32 28 113.

In diesen Schriften offenbarte Gelenkpfannen weisen alle zumindest ein (DE-OS 32 28 113), meist aber mehrere (DE-OS 16 16 151, DE-GM 72 40 856) Fixationselemente auf, mittels derer die in die Pfanne des Beckenknochens gepreßte Gelenkpfanne in dem Beckenknochen verankert werden. Im Falle der DE-OS 32 28 113 ist die Außenfläche mit einem offenzelligen metallischen Belag versehen, in welchen nach der Implantation Knochenbälkchen einwachsen können, wodurch ein sicherer Halt der Gelenkpfanne im Beckenknochen erzielt werden soll. Hernach hat das einstückig mit der Gelenkpfanne ausgebildete Fixationselement in Form eines Zapfens, dessen Ende bei der Implantation einen Schwenkmittelpunkt der Gelenkpfanne bildet, um so ein regelrechtes Kippmoment um seine Achse zu ermöglichen, keine Relevanz mehr.

Andere Gelenkpfannen (beispielsweise gemäß der DE-OS 16 16 151) werden in einem selbsthärtenden Harz zwischen Beckenknochen und ihrer Außenfläche eingebettet zur Erzielung eines Haltes am Knochen.

Die vorerwähnten Konstruktionen eignen sich in gewissen Grenzen für den Ersatz der natürlichen Gelenkpfanne. Voraussetzung für ihren Einsatz ist allerdings stets, daß der Beckenknochen intakt ist, da andernfalls die Fixationselemente keinen Angriffspunkt finden. Dies wird aber bei einer Fülle von zu stellenden Indikationen nicht der Fall sein. So werden beispielsweise bei einem von Knochentumor befallenen Beckenknochen Teile dessen zu resizieren sein, so daß die Implantation bekannter Gelenkpfannenimplantate nicht möglich sein wird.

Aufgabe der vorliegenden Erfindung ist es vor dem aufgezeigten Hintergrund, einen Implantationssatz gemäß dem Oberbegriff des Anspruches 1 anzugeben, aus dem je nach Indikation ein individuell zusammengestelltes Implantat erstellbar ist. Der Implantationssatz soll es also gestatten, ein Implantat auch und insbesondere für einen weiten Indikationsbereich zusammenzustellen.

Gelöst wird diese Aufgabe durch die Ausbildung des Implantationssatzes gemäß dem kennzeichnenden Teil des Anspruches 1.

Stets sind demgemäß mindestens zwei Fixationselemente für die Gelenkpfanne vorgesehen. Mindestens ein erstes Fixationselement greift durch einen als Längsschlitz ausgebildeten Durchgriff in der Gelenkpfanne, beispielsweise durch einen parallel zum Äquator der Kalotte der Gelenkpfanne verlaufenden Längsschlitz, hindurch und ist dort verschieblich geführt. Diese Führung gestattet es, das oder die ersten Fixationselemente zur Auffindung einer als Widerlager (noch) geeigneten Stelle im Beckenknochen in dem Durchgriff dorthin zu führen und anschließend in den Knochen zu treiben. Vorteilhafterweise finden Knochenschrauben Verwendung als erste(s) Fixationselement(e).

Die Gelenkpfanne des Implantationssatzes weist aber darüberhinaus eine weitere Fixationsmöglichkeit für ein oder mehrere zweite Fixationselemente auf. Diese besteht aus einer auf der Außenfläche der Gelenkpfanne vorgesehene, von der metallischen offenzelligen Struktur umfaßte Montageleiste mit mindestens zwei Fixationslagern, in welche wahlweise ein oder mehrere der zweiten Fixationselemente einsetzbar sind.

Der Implantationssatz besteht mithin also stets aus einer Gelenkpfanne und lösbar mit ihr verbindbaren Fixationselementen,die den individuell erforderlichen Umständen vor oder während der Implantation mit der Gelenkpfanne verbunden werden. Eine große Indikationsbreite wird demnach durch den Implantationssatz abgedeckt. Dementsprechend ist ein Erfordernis einer kostenintensiven Lagerhaltung von verschiedenen Formen der Implantate oder aber einer erst nach der Indikation erfolgenden Spezialanfertigung nicht mehr gegeben.

Um die Einbaugröße der Gelenkpfanne durch die auf ihrer Außenfläche vorgesehene Montageleiste nicht zu erhöhen, entspricht deren radiale Ausdehnung vorteilhafterweise höchstens der Dicke der offenzelligen metallischen Struktur auf der Außenfläche der Gelenkpfanne.

Besonders einfach ist die Montage der zweiten Fixationselemente, wenn die Fixationslager in der Montageleiste als Gewindebohrungen ausgebildet sind, an bzw. in welche die zweiten Fixationselemente schraubbar sind. Ob die Fixationselemente an oder in die Gewindebohrungen schraubbar sind, hängt von ihrer Ausbildung ab, wie anhand der nachstehenden Ausführungen erläutert wird.

In die Gewindebohrungen schraubbar sind zweite Fixationselemente, die als mit einem Gewinde versehene Zapfen ausgebildet sind. Operativ geht man in diesem Falle so vor, daß nach der Auffindung geeigneter Widerlager im Beckenknochen für die Fixationselemente Bohrungen in dem Beckenknochen angebracht werden, die in ihrem Durchmesser in etwa dem Durchmesser der beschriebenen Zapfen entsprechen. Die Zapfen werden in die Bohrungen im Beckenknochen gesetzt. Eine weitere Verspannung der Gelenkpfanne in dem Beckenknochen wird erreicht durch Anbringung des oder der ersten Fixationselemente.

Die im übrigen bekannte offenzellige metallische Struktur an der Außenfläche der Gelenkpfanne ermöglicht das Einwachsen von Knochenbälkchen, so daß die Pfanne zementlos in einem äußerst sicheren Sitz im Beckenknochen gehalten wird.

Vorteilhafterweise weisen die als Zapfen ausgebildeten zweiten Fixationselemente einen zentral verlaufenden Einfädelungskanal auf. Durch diesen Einfädelungskanal kann beispielsweise ein Faden geführt werden, mit Hilfe dessen die Heranführung der Zapfen an die Gewindebohrungen in der Montageleiste an der Gelenkpfanne erheblich erleichtert wird.

Andere zweite Fixationselemente sind als Winkelelemente ausgebildet. Diese weisen in einem ersten, dem Krümmungsverlauf der Montageleiste entsprechend konkav ausgebildeten Schenkel ein parallel zu seiner Hauptachse ausgerichtetes Langloch auf, durch das eine Schraube in eine der Gewindebohrungen der Montageleiste in der Gelenkpfanne setzbar ist. In dem anderen Schenkel des Winkelelementes ist eine Bohrung vorgesehen, durch welche eine Knochenschraube setzbar ist. Je nach Indikation können ein oder mehrere dieser Winkelelemente an der Montageleiste montiert werden. Sie gestatten es, etwaig resiziertes Knochenmaterial zu überbrücken, so daß die Gelenkpfanne auch in derartig gelagerten Fällen einen sicheren Halt am Beckenknochen erfahren kann.

An ihren Außenflächen, das heißt also an den im montierten Zustand der Kalotte der Gelenkpfanne abgewandten Flächen, sind die Schenkel der Winkelelemente vorteilhafterweise mit einer offenzelligen metallischen Struktur versehen.

Diese gestattet die Fixation der Winkelelemente im implantiertem Zustand durch das Einwachsen von Knochenmaterial.

Gemäß einer weiteren Ausgestaltung ist das zweite Fixationselement des Implantationssatzes als Winkelelement ausgebildet, das in einem ersten, dem Krümmungsverlauf der Montageleiste entsprechend ausgebildeten Schenkel mehrere parallel ausgerichtete Langlöcher aufweist, durch welche Schrauben in Gewindebohrungen der Montageleiste setzbar sind. In seinem zweiten Schenkel weist dieses Winkelelement Bohrungen auf, durch welche Knochenschrauben setzbar sind.

Ebenso wie die oben erwähnten Winkelelemente mit nur einem Langloch ist das vorerwähnte Winkelelement vorteilhafterweise an seinen Außenflächen, das heißt also wieder an den im montierten Zustand der Kalotte der Gelenkpfanne abgewandten Flächen mit einer offenzelligen metallischen Struktur versehen, in welche zur Erzeugung eines sicheren Haltes Knochenmaterial einwachsen kann.

Das vorerwähnte Winkelelement weist größere Abmessungen auf als die Winkelelemente mit nur einem Langloch. Dies bedeutet, daß mit diesem größeren Winkelelement die Überbrückung noch größerer etwaig resizierter Knochenbereiche möglich sein wird.

Die Erfindung wird nun anhand der Zeichnungen näher erläutert. Hierbei zeigt:
- Figur 1: eine Aufsicht auf die Außenfläche der Gelenkpfanne des Implantationssatzes mit einem Teilschnitt im Bereich der Montageleiste,
- Figur 2: eine Schnittansicht sowie eine Teilansicht der Gelenkpfanne aus Figur 1,
- Figur 3: eine erste Ausführungsform des zweiten Fixationselementes im Längsschnitt (a) und Querschnitt (b),
- Figur 4: eine zweite Ausführungsform des zweiten Fixationselementes in Ansicht (a), Querschnitt (b) und Aufsicht (c),
- Figur 5: eine dritte Ausführungsform des zweiten Fixationselementes in Ansicht (a), Querschnitt (b) und Aufsicht (c),
- Figur 6: eine Schnittansicht eines ersten aus dem Implantationssatz erstellten Implantates, im Beckenknochen implantiert,
- Figur 7: die Ansicht des Implantates aus Figur 6 mit Teilschnittansichten im Bereich der Fixationselemente,
- Figur 8: eine perspektivische Ansicht des Implantates aus den Figuren 7 und 8,
- Figur 9: eine Schnittansicht eines zweiten aus dem Implantationssatz erstellten Implantates, im Beckenknochen implantiert,
- Figur 10: die Ansicht des Implantates aus Figur 9 mit Teilschnittansichten im Bereich der Fixationselemente,
- Figur 11: eine perspektivische Ansicht des Implantates aus den Figuren 9 und 10,
- Figur 12: eine Schnittansicht eines dritten aus dem Implantationssatz erstellte Implantates, im Beckenknochen implantiert,
- Figur 13: die Ansicht des Implantates aus Figur 12 mit Teilschnittansichten im Bereich der Fixationselemente, und
- Figur 14: eine perspektivische Ansicht des Implantates aus den Figuren 12 und 13.

Nachfolgend sind gleich Teile mit denselben Bezugszeichen bezeichnet.

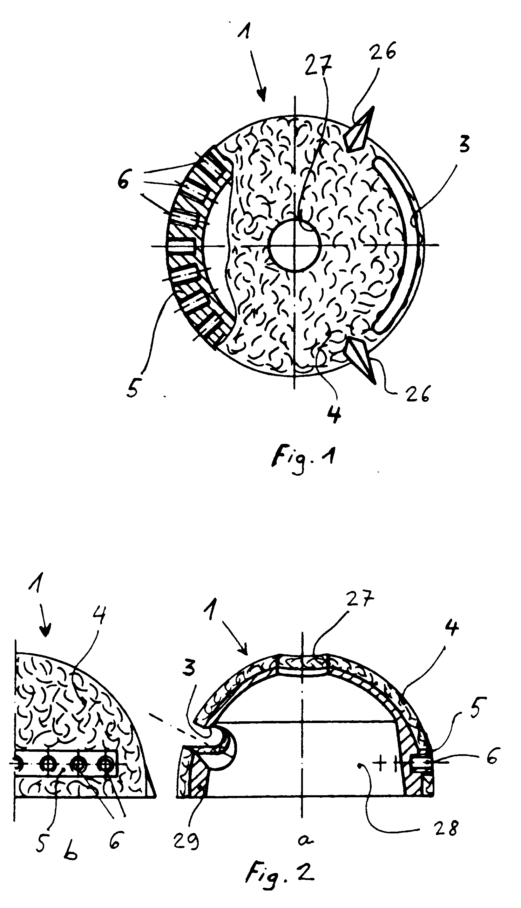

Figur 1 zeigt die Gelenkpfanne des Implantationssatzes in Aufsicht. Die Gelenkpfanne 1 ist kalottenförmig ausgebildet. Im Bereich ihres Poles weist sie eine Bohrung 27 auf, durch welche der Operateur den Abstand der Gelenkpfanne 1 vom Beckenknochen ertasten kann.

Die Gelenkpfanne 1 ist auf ihrer Außenfläche mit einer offenzelligen metallischen Struktur 4 versehen, in welche in bekannter Weise nach der Implantation Knochenmaterial einwachsen kann.

Die Gelenkpfanne 1 weist einen Durchgriff auf, der hier als parallel zum Äquator der Kalotte verlaufender Längsschlitz 3 dargestellt ist. Durch diesen Längsschlitz 3 ist ein erstes Fixationselement setzbar, wie weiter unten näher beschrieben wird.

An der Außenfläche der Gelenkpfanne 1 ist eine Montageleiste 5 dargestellt, die eine Reihe von Fixationslager, hier als Gewindebohrungen 6 dargestellt, aufweist. An bzw. in diese Gewindebohrungen sind zweite Fixationselemente schaubbar, wie weiter unten beschrieben wird. Die Montageleiste 5 ist von der metallischen offenzelligen Struktur 4 umfaßt und schließt mit dieser in allen dargestellten Ausführungsbeispielen bündig ab.

An die Gelenkpfanne 1 sind im übrigen Arretierungskeile 26 angeformt, welche während der Implantation in den Knochen eingetrieben werden und damit eine erste Sicherheit gegen ein Verdrehen der Gelenkepfanne gewährleistet.

Figur 2 zeigt eine Schnittansicht (a) sowie eine Teilansicht (b) der Gelenkpfanne aus Figur 1. Deutlich erkennbar aus der Schnittansicht ist, daß die Gelenkpfanne 1 aus einem massiven Kern 29 und aus der auf diesen aufgebrachten offenzelligen metallischen Struktur 4 aufgebaut ist. Der Kern 29 wird in der Regel aus Metall bestehen. Er schließt einen Raum 28 ein, in dem ein Inlet aus Polyäthylen oder Silikon (nicht dargestellt) als reibungsarmes Lager für die Gelenkkugel (nicht dargestellt) eingesetzt werden kann.

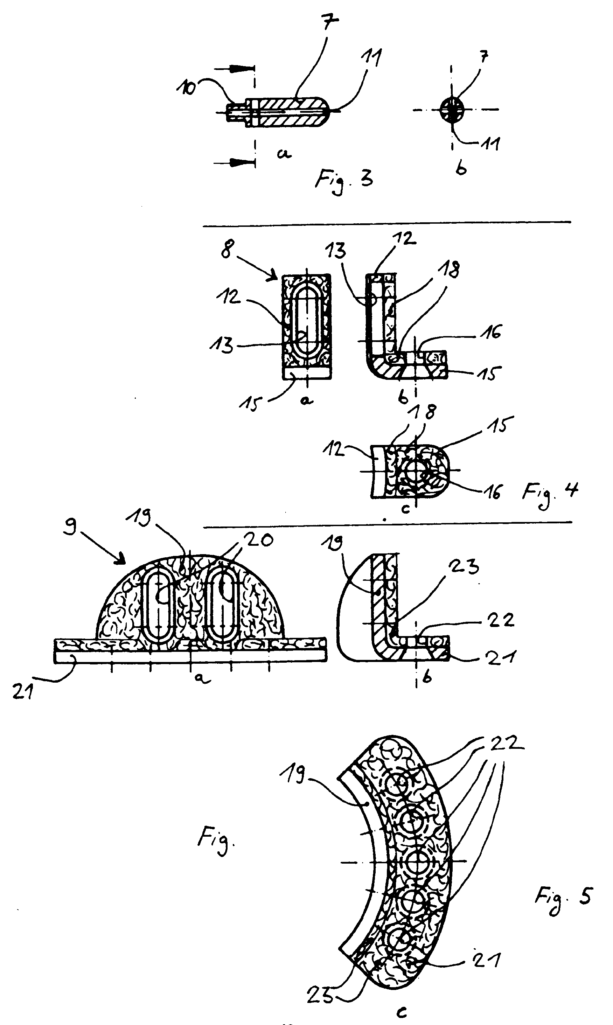

In Figur 3 ist eine erste Ausführungsform des zweiten Fixationselementes 7 in Längsschnitt (a) und Querschnitt (b) dargestellt. Es besteht aus einem Zapfen, das an seinem einen Ende mit einem Gewinde 10 versehen ist,welches in eine Gewindebohrung 6 in der Montageleiste 5 der Gelenkpfanne 1 geschraubt werden kann. Der Zapfen wird in aller Regel aus Metall bestehen, wenn auch grundsätzlich ein geeignetes Kunststoffmaterial in Betracht kommen kann. Der Zapfen weist im dargestellten Ausführungsbeispiel einen zentral verlaufenden Einfädelungskanal 11 auf, mit dessen Hilfe in an sich bekannter Weise die Einführung des Zapfens in eine Gewindebohrung 6 beispielsweise unter Zuhilfenahme eines Fadens erleichtert wird.

In Figur 4 ist eine zweite Ausführungsform des zweiten Fixationselementes 8 dargestellt. Es besteht aus einem Winkelelement mit zwei Schenkeln 12, 15. Der Schenkel 12 ist dem Krümmungsverlauf der Montageleiste 5 entsprechend konkav ausgebildet. In ihm ist ein Langloch 13 vorgesehen, durch welches eine Schraube in eine Gewindebohrung der Montageleiste 5 setzbar ist. Das Langloch 13 ist parallel zu der Hauptachse des Schenkels 12 ausgerichtet. Diese Führung gestattet es, das Fixationselement 8 an der Gelenkpfanne 1 zu verschieben, um ein geeignetes Widerlager im Beckenknochen zu erreichen. Das Fixationselement 8 weist in seinem zweiten Schenkel 15 eine Bohrung 16 auf, durch die eine Knochenschraube setzbar ist. Die im implantierten Zustand dem Knochen zugewandten Flächen des Fixationselementes 8 sind im gezeigten Ausführungsbeispiel mit einer offenzelligen metallischen Struktur 18 versehen, in welche Knochenmaterial einwachsen kann.

Figur 5 zeigt eine dritte Ausführungsform des zweiten Fixationselementes 9. Es ist als Winkelelement ausgebildet, dessen einer Schenkel 19 dem Krümmungsverlauf der Montageleiste 5 der Gelenkpfanne 1 entsprechend konkav ausgebildet ist. In ihm sind mehrere parallel ausgerichtete Langlöcher 20 vorgesehen, durch die Schrauben in die Gewindebohrungen 6 der Montageleiste 5 setzbar sind. Diese Langlöcher 20 gestatten wie schon das Langloch 13 des vorbeschriebenen Fixationselementes 8 die individuelle Einstellung der Lage des Fixationselementes 9 in bezug auf die Gelenkpfanne 1, um ein geeignetes Widerlager im Knochen erreichen zu können. Dieses Widerlager wird benötigt für Knochenschrauben, die durch Bohrungen 22 in dem anderen Schenkel 21 des Fixationselementes 9 setzbar sind. Auch in diesem Ausführungsbeispiel sind die im implantiertem Zustand dem Knochen zugewandten Flächen des Fixationselementes 9 mit einer offenzelligen metallischen Struktur 23 versehen.

Aufgrund seiner Größe ist insbesondere das Fixationselement 9 geeignet, großflächige resizierte Knochenteile zu überbrücken.

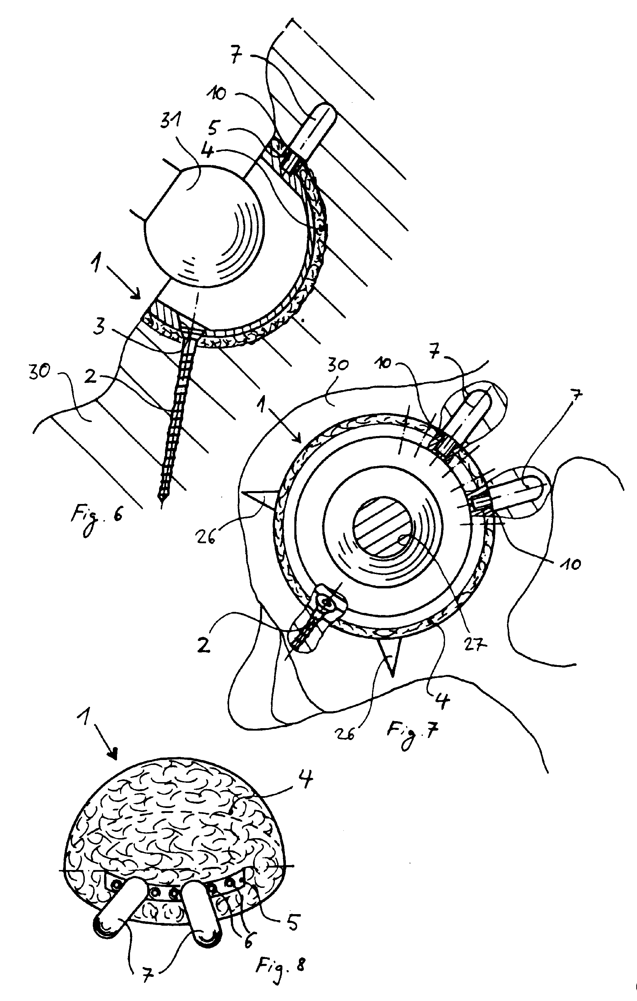

In Figur 6 ist nun eine Schnittansicht eines ersten aus dem Implantationssatz erstellten Implantates, im Beckenknochen 30 implantiert, dargestellt.

Das Implantat besteht in diesem Falle aus der beschriebenen Gelenkpfanne 1, einem ersten Fixationselement 2 in Form einer Knochenschraube, die durch den Durchgriff 3 in der Gelenkpfanne 1 greift und in den Beckenknochen 30 geschraubt ist, und aus zwei zweiten Fixationselementen 7 in Form des beschriebenen Zapfens. Letztere sind in Bohrungen im Beckenknochen 30 gesetzt und mit ihrem Gewinde in Gewindebohrungen 6 in der Montageleiste 5 der Gelenkpfanne 1 verschraubt.

Die Figuren 7 und 8 veranschaulichen dieses Implantat in Aufsicht (Figur 7) und in perspektivischer Ansicht (Figur 8).

Dieses so erstellte Implantat kann bei Indikationen implantiert werden, bei der der Beckenknochen 30 weitestgehend intakt ist.

Im übrigen ist ein Gelenkkopf 31 eines Hüftgelenkes angedeutet, welcher mit der Gelenkpfanne 1 das Gelenk bilden wird.

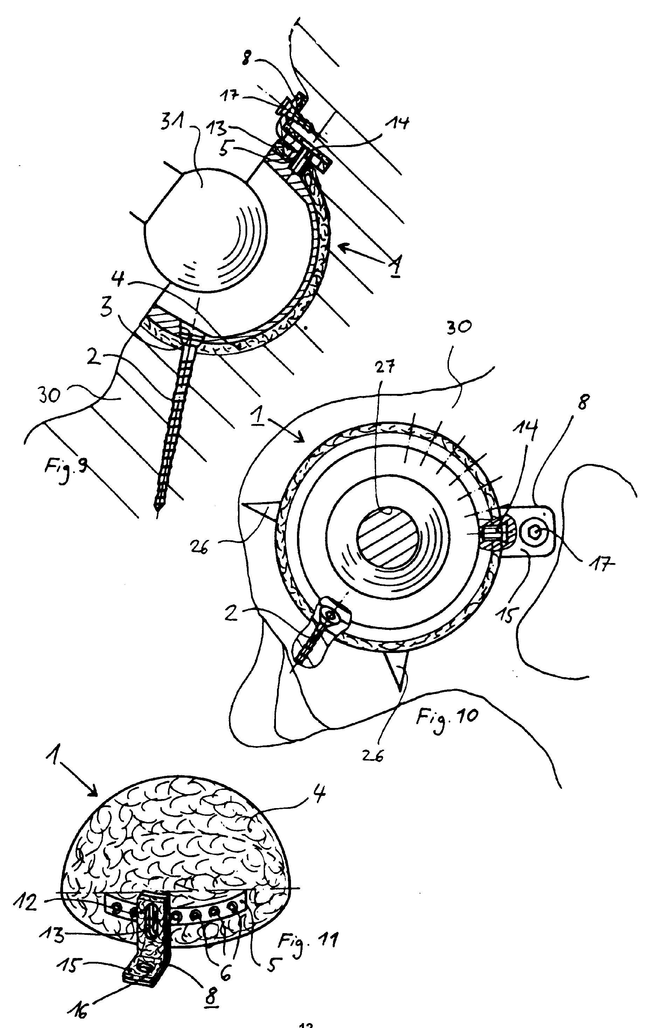

In Figur 9 ist eine der Figur 6 entsprechende Schnittansicht durch ein zweites aus dem Implantationssatz erstelltes Implantat dargestellt.

Das Implantat besteht in diesem Falle aus der beschriebenen Gelenkpfanne 1, einem ersten Fixationselement 1 in Form einer Knochenschraube, die durch den Durchgriff 3 in der Gelenkpfanne 1 greift und in den Beckenknochen 30 geschraubt ist, und aus einem zweiten Fixationselement 8 in Form des beschriebenen Winkelelementes mit einem Langloch 13 im Schenkel 12. Das Winkelelement ist an die Montageleiste 5 der Gelenkpfanne 1 mittels einer Schraube 14 befestigt. Durch die Bohrung 16 im zweiten Schenkel 15 des Winkelelementes ist eine Knochenschraube 17 geführt, mit Hilfe derer das Implantat an dieser Stelle am Beckenknochen 30 fixiert ist.

Mit Hilfe des Langloches 13 in Verbindung mit der Schraube 14 ist es möglich, das Winkelelement vor der Fixation so zu verschieben, daß im Falle der entsprechenden Indikation ein geeignetes Widerlager für die Knochenschraube 17 im Beckenknochen 30 aufgefunden werden kann.

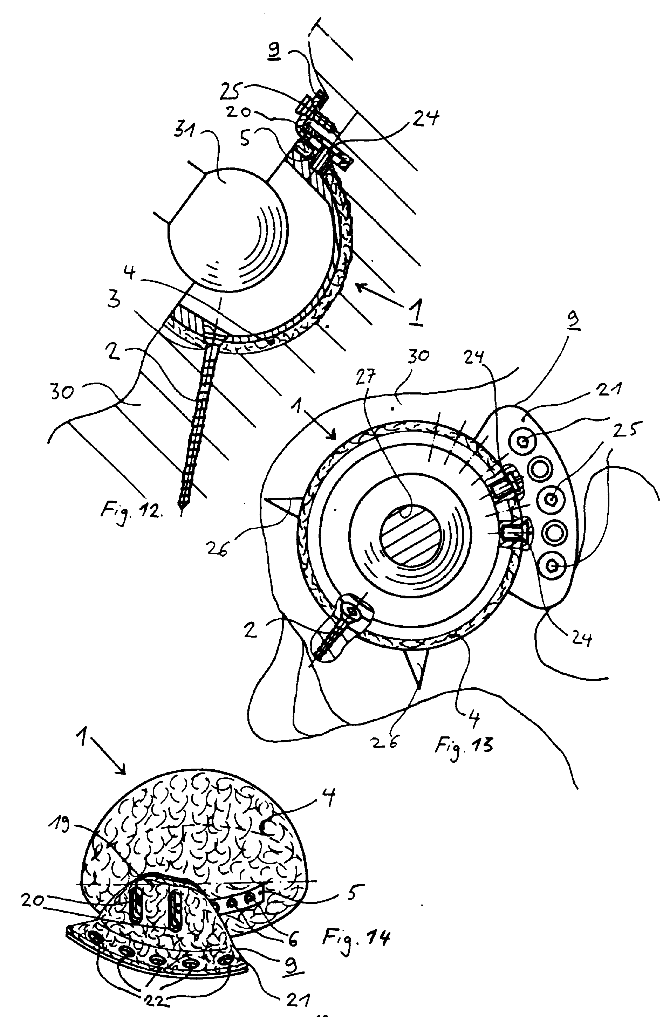

Die Figuren 10 und 11 veranschaulichen dieses Implantat in Aufsicht (Figur 10) und in perspektivischer Ansicht (Figur 11). Aus der letzteren wird in besonders anschaulicher Weise deutlich, welchen Zweck die konkave Ausbildung des Schenkels 12 des Winkelelementes hat. Diese ist notwendig, um eine geeignete Anlagefläche an der Montageleiste 5 in der Gelenkpfanne 1 zu erzielen.

In Figur 12 ist eine den Figuren 6 und 9 entsprechende Schnittansicht eines dritten aus dem Implantationssatz erstellten Implantates dargestellt.

Das Implantat besteht in diesem Falle aus der beschriebenen Gelenkpfanne 1, einem ersten Fixationselement 2 in Form einer Knochenschraube,die durch den Durchgriff 3 in der Gelenkpfanne 1 greift und in den Beckenknochen 30 geschraubt ist, und aus einem zweiten Fixationselement 9 in Form des oben beschriebenen Winkelelementes mit mehreren parallel zueinander ausgerichteten Langlöchern 20 im konkav ausgebildeten Schenkel 19. Durch die Langlöcher 20 greifen Schrauben 24, welche mit ihrem Gewinde in Gewindebohrungen 6 der Montageleiste 5 der Gelenkpfanne 1 eingeschraubt sind. Vor der Fixation des Fixationselementes 9 ist dieses mittels der Langlöcher 20 in bezug auf die Gelenkpfanne 1 längsverschieblich gelagert, so daß im Falle der für die Ausbildung des Implantates in dieser Form gestellten Indikation geeignete Widerlager für Knochenschrauben 25 im Beckenknochen 30 vorliegen werden. Die Knochenschrauben 25 sind durch Bohrungen 22 im zweiten Schenkel des Fixationselementes 9 geführt.

Insbesondere das Fixationselement 9 ist geeignet, insbesondere großflächig resizierte Knochenareale zu überbrücken.

Vorstehend wurden drei Ausführungsbeispiele für aus dem Implantationssatz erstellte Implantate beschrieben. Es ist klar, daß dies nur Beispiele dafür sind, welche Vielzahl von Implantaten aus dem Satz erstellbar sind. Es ist durchaus denkbar, daß beispielsweise nicht nur ein erstes Fixationselement 2 Verwendung findet, sondern auch mehrere. Es können auch mehrere Fixationselemente 8 an die Montageleiste 5 der Gelenkpfanne 1 montiert werden. Die Entscheidung, wie das Implantat im konkreten Einzelfall auszusehen hat, wird von dem Operateur in Abhängigkeit von der gestellten Indikation gefällt werden.

## Patentansprüche

1. Implantationssatz, bestehend aus einer in eine Beckenknochenpfanne einpreßbaren, auf ihrer Außenfläche mit einer offenzelligen metallischen Struktur (4) versehenen kalottenförmigen Gelenkpfanne (1) und aus jeweils mindestens einem ersten (2) und zweiten (7,8,9) Fixationselement zur Verspannung der Gelenkpfanne (1) in dem Beckenknochen (30),
dadurch gekennzeichnet,
daß das erste Fixationselement (2) durch einen als Längsschlitz ausgebildeten Durchgriff (3) in der Gelenkpfanne (1) setzbar und dort verschieblich führbar ist, wobei es radial nach außen weist, und
daß auf der Außenfläche der Gelenkpfanne (1) eine von der metallischen offenzelligen Struktur (4) umfaßte Montageleiste (5) mit mindestens zwei Fixationslagern (6) vorgesehen ist, in welche wahlweise ein oder mehrere zweite Fixationselemente (7,8,9) einsetzbar sind.

2. Implantationssatz nach Anspruch 1, dadurch gekennzeichnet, daß der Durchgriff (3) für das erste Fixationselement (2) in Form einer Knochenschraube ein parallel zum Äquator der Kalotte verlaufender Längsschlitz in der Kalotte ist.

3. Implantationssatz nach Anspruch 1 oder 2, dadurch gekennzeichnet, daß die radiale Ausdehnung der Montageleiste (5) höchstens der Dicke der offenzelligen metallischen Struktur (4) entspricht.

4. Implantationssatz nach einem der Ansprüche 1 bis 3, dadurch gekennzeichnet, daß die Fixationslager (6) der Montageleiste (5) Gewindebohrungen sind, an bzw. in welche die zweiten Fixationselemente (7,8,9) schraubbar sind.

5. Implantationssatz nach Anspruch 4, dadurch gekennzeichnet, daß die zweiten Fixationselemente (7) als mit einem in die Gewindebohrungen der Montageleiste (5) schraubbaren Gewinde (10) versehene Zapfen ausgebildet sind.

6. Implantationssatz nach Anspruch 5, dadurch gekennzeichnet, daß die Zapfen einen zentral verlaufenden Einfädelungskanal (11) aufweisen.

7. Implantationssatz nach Anspruch 4, dadurch gekennzeichnet, daß die zweiten Fixationselemente (8) als Winkelelemente ausgebildet sind, die in einem ersten, dem Krümmungsverlauf der Montageleiste (5) entsprechend konkav ausgebildeten Schenkel (12) ein parallel zu seiner Hauptachse ausgerichtetes Langloch (13) aufweisen, durch welches eine Schraube (14) in eine Gewindebohrung der Montageleiste (5) setzbar ist, und die in dem zweiten Schenkel (15) eine Bohrung (16) aufweisen, durch welche eine Knochenschraube (17) setzbar ist.

8. Implantationssatz nach Anspruch 7, dadurch gekennzeichnet, daß im montierten Zustand der Winkelelemente (8) deren der Kalotte abgewandten Flächen der Schenkel (12,15) mit einer offenzelligen metallischen Struktur (18) versehen sind.

9. Implantationssatz nach Anspruch 4, dadurch gekennzeichnet, daß das zweite Fixationselement (9) als Winkelelement ausgebildet ist, welches in einem ersten, dem Krümmungsverlauf der Montageleiste (5) entsprechend konkav ausgebildeten Schenkel (19) mehrere parallel ausgerichtete Langlöcher (20) aufweist, durch welche Schrauben (24) in Gewindebohrungen der Montageleiste (5) setzbar sind, und welches in dem zweiten Schenkel (21) Bohrungen (22) aufweist, durch die Knochenschrauben (25) setzbar sind.

10. Implantationssatz nach Anspruch 9, dadurch gekennzeichnet, daß im montiertem Zustand des Winkelelementes (9) dessen der Kalotte abgewandten Flächen der Schenkel (19,21) mit einer offenzelligen metallischen Struktur (23) versehen ist.

## Claims

1. Implantation set comprising a calotte-shaped acetabulum (1) that can be forced into a pelvic bone socket and being provided on its outer surface with a metallic open-cell structure (4), and further providing in each case at least a first (2) and a second (7, 8, 9) fixation element to brace the acetabulum (1) in the pelvic bone (30),
characterized in
that the said first fixation element (2) can be placed in the acetabulum (1) through a gap (3) formed as longitudinal slit and there being slidingly guidable in vertically pointing outwards, and in that on the outer surface of the acetabulum (1) a mounting (5) is provided and closed by the said metallic open-cell structure (4) and having at least two fixation bearings (6) into which there can be optionally inserted one or a plurality of the said second fixation elements (7, 8, 9).

2. Implantation set according to claim 1, characterized in that the gap (3) for the said first fixation element (2) in the form of a bone-screw is a longitudinal slit in the said calotte running in parallel to the equator of the said calotte.

3. Implantation set according to claims 1 or 2, characterized in that the radial dimension of the said mounting (5) corresponds to the thickness of the metallic open-cell structure (4) at most.

4. Implantation set according to one of claims 1-3, characterized in that the fixation bearings (6) of the mounting (5) are tapped holes at or into which the said second fixation elements (7, 8, 9) can be screwed.

5. Implantation set according to claim 4, characterized in that the said second fixation elements (7) are developed as pegs provided with a thread that can be screwed into the tapped holes of the mounting (5).

6. Implantation set according to claim 5, characterized in that the said pegs comprise a centrally running threading channel (11).

7. Implantation set according to claim 4, characterized in that the said second fixation elements (8) are developed as angle elements comprising a longitudinal hole (13) orientated in parallel to its major axis in a first leg dished in accordance with the course of the curvature of the mounting (5), through which longitudinal hole a screw (14) is placeable into a tapped hole of the mounting (5) and which angle elements further comprise a hole (16) in the said second leg (15) through which a bone-screw (17) can be placed.

8. Implantation set according to claim 7, characterized in that, the angle elements (8) being mounted, the areas of the legs (12, 15) turned away from the calotte are provided with a metallic open-cell structure (18).

9. Implantation set according to claim 4, characterized in that the second fixation element (9) is developed as an angle element comprising a plurality of longitudinal holes (20) aligned in parallel and dished in accordance with the course of the curvature of the mounting (5), through which longitudinal holes screws (24) are placeable into tapped holes of the said mounting (5) and which angle element comprises holes (22) in the said second leg (21) through which the bone-screws (25) can be placed.

10. Implantation set according to claim 9, characterized in that, the angle element (9) being mounted, the areas of the legs (19, 21) turning away from the calotte are provided with a metallic open-cell structure (23).

## Revendications

1. Ensemble d'implantation, composé d'une cupule d'articulation (1) en forme de calotte, susceptible d'être enfoncée dans une cavité osseuse du bassin, pourvue sur sa surface extérieure d'une structure métallique (4) à cellules ouvertes et d'au moins un premier (2) et d'un deuxième (7,8,9) éléments de fixation, destinés à enserrer la cupule d'articulation (1) dans l'os du bassin (30),
caractérisé en ce que
le premier élément de fixation (2) est insérable, à travers un passage (3), réalisé sous forme de fente allongée, dans la cuvette d'articulation (1) et est susceptible d'y être guidé en translation, en étant tourné radialement vers l'extérieur, et
en ce que, sur la surface extérieure de la cupule d'articulation (1), est prévue une bande de montage (5), entourée par la structure métallique (4) à cellules ouvertes, avec au moins deux points de fixation (6), dans lesquels au choix un ou plusieurs deuxièmes éléments de fixation (7,8,9) peuvent être insérés.

2. Ensemble d'implantation selon la revendication 1, caractérisé en ce que le passage (3) destiné au premier élément de fixation (2), se présentant sous la forme d'une vis à os, est une fente allongée, s'étendant parallèlement à l'équateur de la calotte.

3. Ensemble d'implantation selon la revendication 1 ou 2, caractérisé en ce que l'étendue radiale de la bande de montage (5) correspond tout au plus à l'épaisseur de la structure métallique (4) à cellules ouvertes.

4. Ensemble d'implantation selon l'une des revendications 1 à 3, caractérisé en ce que les points de fixation (6) de la bande de montage (5) sont des trous taraudés, sur lesquels, ou bien dans lesquels les deuxièmes éléments de fixation (7,8,9) sont susceptibles d'être vissés.

5. Ensemble d'implantation selon la revendication 4, caractérisé en ce que les deuxièmes éléments de fixation (7) sont réalisés sous forme de tourillons, pourvus d'un filetage (10) susceptible d'être vissé dans les trous taraudés de la bande de montage (5).

6. Ensemble d'implantation selon revendication 5, caractérisé en ce que les tourillons présentent un canal d'enfilage (11) s'étendant centralement.

7. Ensemble d'implantation selon la revendication 4, caractérisé en ce que les deuxièmes éléments de fixation (8) sont réalisés sous forme d'éléments en équerre, présentant, dans une première branche (12), concave et de réalisation correspondant à l'allure incurvée de la bande de montage (5), un trou allongé (13) orienté parallèlement à son axe principal, au travers duquel une vis (14) peut être insérée dans un trou taraudé de la bande de montage (5), les deuxièmes éléments de fixation (8) présentant dans la deuxième branche (15) un trou (16) à travers lequel on peut faire passer une vis à os (17).

8. Ensemble d'implantation selon la revendication 7, caractérisé en ce qu'à l'état monté des éléments en équerre (8), les surfaces, opposées à la calotte, de leurs branches (12,15) sont pourvues d'une structure métallique (18) à cellules ouvertes.

9. Ensemble d'implantation selon la revendication 4, caractérisé en ce que le deuxième élément de fixation (9) est réalisé sous forme d'élément en équerre, présentant, dans une première branche (19), concave et de réalisation correspondant à l'allure incurvée de la bande de montage (5), plusieurs trous allongés (20) parallèles, au travers desquels des vis (24) peuvent être insérées dans des trous taraudés de la bande de montage (5), le deuxième élément de fixation (9) présentant dans la deuxième branche (21) des trous (22) à travers lequel on peut faire passer des vis à os (25).

10. Ensemble d'implantation selon la revendication 9, caractérisé en ce qu'à l'état monté de l'élément en équerre (9), les surfaces, opposées à la calotte, de ses branches (19, 21) sont pourvues d'une structure métallique (23) à cellules ouvertes.
